# EUROPÄISCHE PATENTANMELDUNG

(11) **EP 1 279 416 A2**
(43) Veröffentlichungstag der Anmeldung: **29.01.2003**
(21) Anmeldenummer: 02015793.9
(22) Anmeldetag: 15.07.2002
(51) Int. Cl.: A63B 24/00, A61B 5/00

(54) **Analysesystem zur Trainingskontrolle in der Rehabilitation**

(30) Priorität: 27.07.2001 DE 10136759
(71) Anmelder: SIEMENS AKTIENGESELLSCHAFT, 80333 München (DE)
(72) Erfinder: Abraham-Fuchs, Klaus, 91058 Erlangen (DE); Eisermann, Uwe, 91052 Erlangen (DE); Schmidt, Kai-Uwe, Dr., 91052 Erlangen (DE)

(57) **Zusammenfassung**

Analysesystem zur Kontrolle und Optimierung eines von einem Patienten ohne ständige Betreuung durchzuführenden Trainingsprogramms, mit an den Trainingsgeräten und/oder am Patienten und/oder in einem Patienten-PC angebrachte computergestützte Messmodule zur Erfassung der Aktivität und/oder des Bewegungsablaufs des Patienten zur Ableitung von Kenngrößen für die Patienten-Compliance.

## Beschreibung

Die Erfindung bezieht sich auf ein Analysesystem zur Kontrolle und Optimierung eines von einem Patienten ohne ständige Betreuung durchzuführenden Trainingsprogramms.

Eine wesentliche Komponente der Rehabilitation ist das Eigentraining des Patienten, das heißt das eigenständige, eigenverantwortliche und in der Regel ohne aktive Betreuung durch den Arzt oder Therapeuten erfolgende Training des Patienten zur Verbesserung rehabilitationsrelevanter Fähigkeiten. Bei diesem Training handelt es sich in Abhängigkeit von Diagnose(n) und Indikation(en) um ein Training motorischer Fähigkeiten (z. B. Kraft, Beweglichkeit, Gleichgewichtsfähigkeit) und/oder kognitiver Fähigkeiten (Aufmerksamkeit, Gedächtnis). So werden in der orthopädischen Rehabilitation (z. B. nach Knochenfrakturen) und in der kardiologischen Rehabilitation (z. B. nach Herzinfarkt) vorrangig motorische Fähigkeiten trainiert. In der neurologischen Rehabilitation (z. B. nach Hirninfarkt/-blutung,. das heißt "Schlaganfall") werden dagegen vorrangig kognitive Fähigkeiten trainiert. Wenn motorische und kognitive Funktionen beeinträchtigt sind, ist ein kombiniertes Training (Training motorischer und kognitiver Fähigkeiten) indiziert. Diese Trainingsform wird unter anderem in der geriatrischen Rehabilitation (z. B. bei Morbus Alzheimer) und auch in der neurologischen Rehabilitation bei komplexen Schädigungen (z. B. Schlaganfall mit Halbseitenlähmung) favorisiert.

In den angesprochenen Bereichen der Rehabilitation werden heute zunehmend innovative computerbasierte Konzepte eingesetzt, beispielsweise der von der Siemens AG für die orthopädische Rehabilitation entwickelte Prototyp "HealthMan Physio Assistant". Der Physio Assistant ist ein Computersystem mit Trainings- und Therapiekontroll- sowie Informations- und Kommunikationsfunktionen. Das System besteht aus mindestens einem Experten-Terminal ("Experten-Client") für den Arzt oder Therapeuten, einem zentralen oder dezentralen Server und je nach Architektur aus einem oder mehreren Patienten-Terminals ("Patienten-Client/s"). Den Experten-Client (PC) des Physio Assistenten verwendet der medizinische Betreuer, um individuell optimale Trainingsprogramme für den Patienten zu erstellen. Diese werden auf telematischem Weg zum Patienten-Client (PC) transferiert. Dieser bereitete die Vorgaben des Arztes oder Therapeuten auf und bietet dem Patienten multimediale Real-Time-Trainingsprogramme.

Entscheidend für den Trainingserfolg ist die "Compliance" des Patienten, das heißt die Einhaltung des verordneten Trainings unter Berücksichtigung der vorgegebenen, auf die individuellen Bedingungen abgestimmten Belastungsfaktoren qualitativer und quantitativer Art (z. B. Dauer und Häufigkeit der Ausführung und Einhaltung vorgegebener Randbedingungen) (vgl. Weineck 1996, S. 24). Um die Compliänce des Patienten durch Interaktion mit dem Patienten und Adaptation des Trainings verbessern zu können, muss der Arzt oder Therapeut wissen

| | |
|---|---|
| • wann und wie lange der Patient trainiert hat | (Zeitaspekt objektiv) |
| • ob der Patient nach den Vorgaben trainiert hat | (Ausführungsaspekt, objektiv) |
| • wie der Patient sich fühlt und einschätzt | (Befindlichkeitsaspekt, subjektiv). |

Die Erfindung hat zum Ziel, die Compliance des Patienten speziell im Kontext der Durchführung von therapeutischen Trainingsprogrammen zu messen. Diese Trainingsprogramme umfassen beispielhaft etwa physiotherapeutische Übungen (Beweglichkeitstraining, Krafttraining etc.), neurologische Übungen (Training von Koordinationsstörungen, Sprachstörungen) oder kardiologische Übungen (Kreislauftraining).

Im Rahmen der Durchführung verordneter medizinischer Übungen können Messgrößen für die Compliance des Patienten z. B. sein:
- die Tatsache, dass der Patient eine Übung überhaupt durchführt (z. B. gemessen durch Einschalten eines Gerätes, wie Computer, Fahrradergometer, Messsignale von Sensoren am Körper des Patienten, Feststellung der Dauer und Häufigkeit des Aufrufens eines Trainingsprogramms im Patientencomputer etc.)
- die Dauer der Durchführung (Einschaltdauer, Dauer des Datenflusses von Daten, die während des Trainings erzeugt werden etc., z. B. auch von Bewegungssensoren am Körper des Patienten oder am Trainingsgerät)
- Messung der körperlichen Bewegung des Patienten (hat er Sensoren zur Messung der Bewegung überhaupt in Betrieb? Lassen die Sensorsignale einen Rückschluss darauf zu, dass das richtige Körperteil bewegt wird? ..dass das richtige Körperteil auf die erwartete Art und Weise bewegt wird?)
- Messung physiologischer Größen, die durch die Durchführung der Übungen verändert werden (z. B. Herzrate, Blutdruck, Sauerstoffsättigung, CO2 in der Atemluft etc.)
- Messung der Befindlichkeit des Patienten, typischer erfasst durch Eintrag in ein "medizinisches Patienten-Tagebuch" (z. B. Erschöpfungszustand, Schmerzen, Schlaflosigkeit etc.)

Je nach Qualität der verwendeten Sensorik und Messaufwand können die erfassten Messwerte zur Quantifizierung der Compliance eine Aussagekraft in drei Qualitätsstufen haben:
- werden Übungen überhaupt ausgeführt, und mit welcher Dauer und Häufigkeit?
- werden die Übungen in einer Art und Weise ausgeführt, dass der erwartete Nutzen der Übung überhaupt erreichbar ist (richtiges Körperteil? eine Belastung des Körpers erkennbar? etc.)
- werden die Übungen im Rahmen voreingestellter Grenzwerte richtig oder falsch ausgeführt? (z. B. Ausmaß, Winkel etc. einer körperlichen Bewegung, Anstieg der Herzfrequenz mindestens um x, aber nicht mehr als y etc.)

Der Arzt oder Therapeut kann diese Aspekte bislang lediglich durch Beobachtung und mündliche Befragung erfassen und das Training den Ausprägungen entsprechend verändern. Diese Vorgehensweise ist zeitintensiv und nicht für jeden Patienten und jede Trainingseinheit realisierbar. Die im Weiteren zu beschreibende Vorrichtung bietet dem Arzt oder Therapeuten die Möglichkeit einer umfassenden Analyse des Trainings des Patienten und seiner Compliance, ohne diesen dabei beobachten und mündlich befragen zu müssen. Sie ist damit generell für eine Vielzahl telemedizinischer Betreuungsformen und speziell für das skizzierte Konzept Physio Assistant ideal geeignet.

Trainings- bzw. Compliance-Analysen sind nicht mit Therapieverlaufskontrollen gleichzusetzen. Für Therapieverlaufskontrollen entwickelte Vorrichtungen werden eingesetzt, um Veränderungen ausgewählter Funktionen im Verlauf der Therapie zu erfassen (z. B. Veränderung des Gangbildes im Laufe eines stationären Reha-Aufenthaltes). Dagegen wird eine Vorrichtung zur Trainings- bzw. Compliance-Analyse verwendet, um die oben genannten trainingsspezifischen Aspekte zu "messen" und dem Arzt oder Therapeuten mitzuteilen, ob und wie der Patient trainiert hat.

All diese Informationen zur Analyse des Trainings und der Compliance des Patienten mit anschließend durchgeführten Maßnahmen zur Verbesserung der Compliance konnte bisher lediglich durch direkte Beobachtung, Befragung durch einen Arzt oder Therapeuten erfolgen.

Aus der US 6,210,301 ist bereits eine Anordnung bekannt geworden, bei der Sensoren am Körper (Beschleunigung, Winkel, Druck, Scherkräfte) die Bewegungsmuster des Patienten erfassen, gegen vorgegebene Werte vergleichen und dem Benutzer ein Biofeedback während der Übung über den Grad der Richtigkeit der Durchführung geben.

Die DE 198 46 982 A1 beschreibt bereits ein Verfahren und System zur Überwachung der Haltung eines Benutzers an einem Trainingsgerät unter Verwendung von rein kinematischen Sensoren am Benutzer oder an Teilen des Trainingsgeräts. Auch hier geht es nur um eine reine Bewegungskontrolle dahingehend, ob der Benutzer das Gerät richtig bedient. Beide vorstehende Patente zielen auf ein Biofeedback zur richtigen Durchführung von Bewegungsabläufen innerhalb eines physiologischen Trainings ab, nicht aber auf eine Messung der Compliance zur Beurteilung für außenstehende Dritte und umfassen auch nur wenige Bewegungsaspekte aber keine häufig notwendigen Messgrößen wie Zeiterfassung, physiologische Parameter oder Befindlichkeiten.

In der DE 197 02 150 A1 ist ein Analysesystem zur Trainingskontrolle in der Rehabilitation beschrieben, bei der also wiederum keine Compliance-Analyse möglich ist, sondern nur eine Therapieverlaufskontrolle stattfindet. Zu diesem Zweck sind Sensoren für Biomedizinische Signale vorhanden, aus denen ein Signalmuster abgeleitet wird, das die Durchführung einer Übung charakterisiert. Dieses Signalmuster wird mit einem Sollmuster für die optimale Übungsdurchführung verglichen, wobei die Vergleichsergebnisse als Biofeedback für den Patienten und als Maß für den Trainingserfolg an den Arzt übermittelt werden. Es geht dabei ausschließlich um eine Therapieverlaufskontrolle. Die "Compliance" des Patienten kann nicht erfasst werden.

Auch die US 5,810,747 beschreibt wiederum ein Teletrainingssystem, mit Therapieverlaufskontrolle. Hierzu ist ein Computerarbeitsplatz für den Patienten vorgesehen, der vernetzt ist mit einem Computerarbeitsplatz für den Arzt, wobei Sensoren (elektrophysiologisch, speziell EMG und/oder motorisch, speziell Goneometer) vorhanden sind und die bei der Durchführung einer Übung registrierten Sensorsignale gesammelt werden. Aus den Sensorsignalen werden Ableitungen einer Modifikation der Übung erstellt, entweder interaktiv durch den Arzt oder durch einen Expertensystem, wobei die modifizierte Übungsanleitung oder Übungsziele zum Patientencomputer übertragen werden. Dabei wird der Übungserfolg quantitativ bewertet und aus diesen Bewertungszahlen eine Modifikationsänderung für die Übung abgeleitet. In einer ersten Ausführungsform ist der Arzt Real-Time mit dem Patienten verbunden und kann die bei der Übungsdurchführung aufgenommenen Signale direkt beobachten. In der zweiten Ausführungsform wird die Qualität der Übungsdurchführung aus den Signalen von einem Expertensystem beurteilt und automatisch Modifikationsvorschläge abgeleitet. Auch dies ist wiederum eine reine Therapieverlaufskontrolle, bei der (abgesehen von dem Ausführungsbeispiel mit Expertensystem) der Arzt ja unmittelbar die Tätigkeit des Patienten überwacht. Hier stellt sich ja die Frage einer Patientencompliance überhaupt nicht.

Die in den beiden vorstehend angesprochenen Druckschriften beschriebenen Verfahren zur Therapie-Verlaufskontrolle können z. B. einen schlechten Trainingserfolg messen und dokumentieren. Aus der Tatsache, dass die Übung mit schlechtem Erfolg durchgeführt wurde, ist jedoch nicht zu erkennen, warum dies der Fall ist. Insbesondere können die beiden Fälle nicht unterschieden werden, ob
das bestehende Defizit des Patienten Ursache für den schlechten Übungserfolg ist, der Patient also einfach weitertrainieren muss,
oder der Patient den Vorgaben zur Durchführung der Übung nicht folgt (zu selten durchführt, zu kurz durchführt, auf falsche Art und Weise durchführt, nicht mit dem notwendigen Einsatz an Kraft und Konzentration durchführt usw.) also ein Mangel an Compliance eher als ein bestehendes Defizit die Ursache ist. In diesem Fall ist weniger die Änderung der Übung sinnvoll, als Maßnahmen zur Verbesserung der Compliance (Gesprächschulung usw.).

Die Compliance ist aber bei beiden vorstehend beschriebenen Systemen überhaupt nicht angesprochen, sodass damit auch eine Unterscheidung und Beurteilung weshalb ein schlechter Übungserfolg vorliegt gar nicht möglich ist.

Der Erfindung liegt daher die Aufgabe zugrunde, ein Analysesystem zu schaffen, welches eine, ohne Einzelbefragung durch einen Arzt oder Therapeuten realisierbare, automatische Kontrolle des Trainings ermöglicht.

Zur Lösung dieser Aufgabe ist ein Analysesystem der eingangs genannten Art erfindungsgemäß gekennzeichnet durch an den Trainingsgeräten und/oder am Patienten und/oder in einem Patienten-PC angebrachte computergestützte Messmodule zur Erfassung der Aktivität und/oder des Bewegungsablaufs des Patienten zur Ableitung von Kenngrößen für die Patienten-Compliance. Unter Trainingsgerät ist dabei - bei einem Training der kognitiven Fähigkeiten - auch ein PC oder Computer mit integriertem Messmodul zu verstehen.

Die Messmodule umfassen als ersten Typ sogenannte Ereignismodule zur Bestimmung zeitbezogener Parameter, wie beispielsweise die Gesamtdauer des Trainings (brutto und netto) und die Dauer der einzelnen Übungen. Das Ereignismodul kann automatisch oder manuell vom Patienten aktiviert werden.

Darüber hinaus sollen Bewegungsmodule zur Bestimmung der vom Patienten ausgeführten Bewegungen vorgesehen sein, was selbstverständlich nur für das Training motorischer Fähigkeiten relevant ist. Die Messungen liefern Informationen darüber, ob der Patient sich überhaupt bewegt hat, ob er das richtige Körperteil bewegt hat, z. B. Vorgabe: linkes Bein bewegen - Patient bewegt linkes Bein: Vorgabe erfüllt. Darüber hinaus kann die Messung auch Informationen darüber liefern, ob er das Körperteil gemäß der Vorgabe bewegt hat, z. B. linkes Bein ganz durchstrecken.

Ein dritter, für das erfindungsgemäße Analysesystem bedeutsamer Typ von Messmodulen sind Messmodule für elektrophysiologische Parameter, wie beispielsweise Herzfrequenz, Blutdruck, Temperatur, Potentialdifferenzen (z. B. EKG, EMG) und Potentialschwankungen (z. B. EEG). Analog zum Bewegungsmessmodul wird für ausgewählte Parameter erfasst, ob sie sich überhaupt verändern (Anstieg der Herzfrequenz beim Trainingsbeginn), ob sie sich so verändern, dass auf Ausführung eines Trainings geschlossen werden kann (z. B. Anstieg und Abfall der Herzfrequenz im Wechsel) oder ob sie sich so verändern, dass auf die Ausführung des vorgegebenen Trainings geschlossen werden kann (z. B. Anstieg und Abfall der Herzfrequenz gemäß Belastungsvorgaben, wie beispielsweise der Belastungsintensität, der Belastungsdauer und der Belastungsdichte).

Schließlich kann in weiterer Ausgestaltung der Erfindung auch noch der Einsatz eines Befindlichkeitsmessmoduls vorgesehen sein, der zu definierten Zeitpunkten Fragen vorgibt, die vom Patienten beantwortet werden müssen, z. B. zum Ablauf des Trainings, zur Anstrengung und zur Befindlichkeit. Derartige Fragebögen können in Anlehnung an bekannte Fragebögen zur Lebensqualität gestaltet werden.

Der Arzt oder Therapeut entscheidet, welche der vier Module für die spätere Analyse der Compliance bzw. einer Trainingseinheit eingesetzt werden soll. Das Ereignismessmodul und/oder Bewegungsmodul und/oder das Messmodul für elektrophysiologische Parameter und/oder das Befindlichkeitsmessmodul.

Die angesprochenen Messmodule können - wenn es sich nicht um reine Bewegungssignale am Trainingsgerät oder Messsignale von Sensoren am Körper des Patienten handelt - direkt in einen Patienten-PC integriert sein, das heißt speziell die Ereignismodule und/oder Befindlichkeitsmodule können Bestandteil einer auf einem Patienten-PC abspielbaren Software sein, wobei eine solche Software auch das oder die Trainingsprogramme beinhalten kann, die aufgrund der Beurteilung der Compliance durch den Arzt von diesem dem Patienten zur Verfügung gestellt werden. Der Patient erhält entweder eine Diskette oder es wird ihm die jeweilige Software mit dem Trainingsprogramm und den Ereignis- oder Befindlichkeitsmodulen online vom Arzt oder Betreuungszentrum auf seinen PC überspielt.

Bevorzugt ist ein erfindungsgemäßes Analysesystem in ein telemedizinisches Behandlungssystem integriert, mit einem zentralen oder dezentralen Server mit Zugangsterminal für den Patienten sowie den Arzt oder Therapeuten.

In weiterer Ausgestaltung der Erfindung können die abgeleiteten Kenngrößen für die Compliance nicht nur an einen zentralen Server, bzw. den Arzt-PC übertragen werden, sondern auch in einer elektronischen Patientenakte zusammen mit anderen Gesundheitsdaten des Patienten gespeichert werden.

Dabei hat es sich weiter als zweckmäßig erwiesen, die abgeleiteten Kenngrößen für die Compliance graphisch darzustellen, insbesondere in ihrem zeitlichen Verlauf oder im Vergleich zwischen der Compliance zu verschiedenen Trainingseinheiten.

Mit Vorteil können aus den Kenngrößen für die Compliance gegebenenfalls medizinisch indizierte Änderungen des Trainingsprogramms abgeleitet werden, wobei das geänderte Trainingsprogramm auf elektronischem Wege direkt an den Patienten übersandt werden kann.

Schließlich liegt es auch noch im Rahmen der Erfindung, bei schlechter Compliance des Patienten ein Alarmsignal abzuleiten, das direkt an den Arzt-PC weitergeleitet wird.

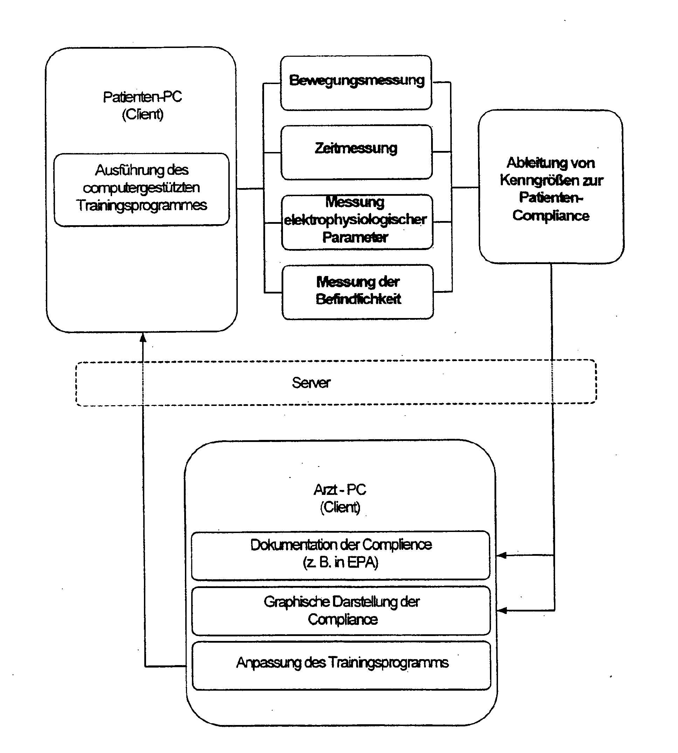

Weitere Vorteile, Merkmale und Einzelheiten der Erfindung ergeben sich aus der nachfolgenden Beschreibung eines Ausführungsbeispiels sowie anhand der Zeichnung, die ein schematisches Ablaufdiagramm eines erfindungsgemäßen Analysesystems im Zuge eines telemedizinischen Behandlungssystems zeigt.

Über den Patienten-PC erfolgt eine Computerunterstützung der verschiedenen Messmodule für die Bewegungsmessung, die Zeitmessung, die Messung elektrophysiologischer Parameter und die Messung der Befindlichkeit. Die zeitbezogenen Parameter können dabei über eine Uhr (Einschalten der Zeitmessung nach Anmeldung am Client und Ausschalten der Zeitmessung nach Abschaltung) über Mausklicks oder über die Trainingsgeräte selbst (Ein- und Ausschalten der Zeitmessung analog zum Einund Ausschalten von Trainingsgeräten) gemessen werden. Eine Bewegung kann über den Bewegungsmessmodul wie folgt gemessen werden: Opto-elektronische Sensoren (z. B. Kamera, Webcam), Beschleunigungssensoren, Winkelmesssensoren, Drucksensoren oder Dehnungssensoren.

Zur Messung der elektrophysiologischen Parameter können Herzfrequenzmessgeräte (z. B. Handgelenkmanschette, Brustgurt) Blutdruckmessgeräte, Thermometer, EKG- und EEG-Geräte eingesetzt werden.

Aus den Messdaten der verschiedenen Messmodule werden Kenngrößen zur Patienten-Compliance abgeleitet und nach Datenfernübertragung zu einem Server auf dem Arzt-PC grafisch aufbereitet. Wichtig ist hier insbesondere die übersichtliche Darstellung der Kenngrößen im zeitlichen Verlauf über die Gesamtdauer der Betreuung, um so Entwicklungstrends darstellen zu können.

Der Arzt bewertete das Ergebnis und sendet dem Patienten Empfehlungen oder ein modifiziertes Trainingsprogramm zu.

Ein entscheidender Vorteil dieser Lösung ist darin zu sehen, dass der Arzt oder Therapeut die zentralen Informationen über das Training erhält, ohne selbst anwesend sein zu müssen. Eine computergestützte Auswertung vorausgesetzt, kann er auf diese Weise mehr Patienten pro Zeiteinheit betreuen und individuell optimal behandeln. Für den Patienten ist ein Vorteil in der optimalen Behandlung zu sehen. Darüber hinaus kann er zeit- und personalunabhängig trainieren. Überdies ist denkbar, ein solcherart fernüberwachtes Training in den teil- und poststationären Bereich zu verlagern, wodurch sich stationäre Aufenthalte verkürzen und Kosten für die Rehabilitation verringern ließen.

## Patentansprüche

1. Analysesystem zur Kontrolle und Optimierung eines von einem Patienten ohne ständige Betreuung durchzuführenden Trainingsprogramms, **gekennzeichnet durch** an den Trainingsgeräten und/oder am Patienten und/oder in einem Patienten-PC angebrachte computergestützte Messmodule zur Erfassung der Aktivität und/oder des Bewegungsablaufs des Patienten zur Ableitung von Kenngrößen für die Patienten-Compliance.

2. Analysesystem nach Anspruch 1, **dadurch gekennzeichnet, dass** die Messmodule Ereignismodule zur Bestimmung zeitbezogener Parameter umfassen.

3. Analysesystem nach Anspruch 1 oder 2, **dadurch gekennzeichnet, dass** die Messmodule Bewegungsmodule zur Bestimmung der vom Patienten ausgeführten Bewegungen umfassen.

4. Analysesystem nach einem der Ansprüche 1 bis 3, **gekennzeichnet durch** Messmodule zur Erfassung der elektrophysiologischen Parameter des Patienten.

5. Analysesystem nach einem der Ansprüche 1 bis 4, **gekennzeichnet durch** ein Befindlichkeitsmodul zur Befragung des Patienten zu subjektiven Gesichtspunkten des Trainings wie Trainingsablauf, Anstrengung, momentane Befindlichkeit oder dergleichen.

6. Analysesystem nach einem der Ansprüche 1 bis 5, **dadurch gekennzeichnet, dass** es in ein telemedizinisches Behandlungssystem integriert ist.

7. Analysesystem nach Anspruch 5, **gekennzeichnet durch** einen zentralen oder dezentralen Server mit Zugangsterminals für den Patienten sowie den Arzt oder Therapeuten.

8. Analysesystem nach einem der Ansprüche 1 bis 7, **dadurch gekennzeichnet, dass** die abgeleiteten Kenngrößen für die Compliance an einen zentralen Server bzw. den Arzt-PC übertragen werden.

9. Analysesystem nach einem der Ansprüche 1 bis 8, **dadurch gekennzeichnet, dass** die abgeleiteten Kenngrößen für die Compliance in einer elektronischen Patientenakte zusammen mit anderen Gesundheitsdaten des Patienten gespeichert werden.

10. Analysesystem nach einem der Ansprüche 1 bis 9, **dadurch gekennzeichnet, dass** die abgeleiteten Kenngrößen für die Compliance graphisch dargestellt werden, insbesondere in ihrem zeitlichen Verlauf oder im Vergleich zwischen der Compliance zu verschiedenen Trainingseinheiten.

11. Analysesystem nach einem der Ansprüche 1 bis 10, **dadurch gekennzeichnet, dass** aus den Kenngrößen für die Compliance gegebenenfalls medizinisch indizierte Änderungen des Trainingsprogramms abgeleitet werden.

12. Analysesystem nach einem der Ansprüche 1 bis 11, **dadurch gekennzeichnet, dass** das geänderte Trainingsprogramm auf elektronischem Wege an den Patienten-PC übersendet wird.

13. Analysesystem nach einem der Ansprüche 1 bis 12, **dadurch gekennzeichnet, dass** bei schlechter Compliance des Patienten ein Alarmsignal abgeleitet und dieses Alarmsignal an den Arzt-PC weitergeleitet wird.

14. Analysesystem nach einem der Ansprüche 1 bis 13, **dadurch gekennzeichnet, dass** die Ereignismodule und/oder Befindlichkeitsmodule Bestandteile einer auf einem Patienten-PC abspielbaren Software sind.

15. Analysesystem nach Anspruch 14, **dadurch gekennzeichnet , dass** die Software auch das Trainingsprogramm enthält.
